(19) **Europäisches Patentamt**

**European Patent Office**

**Office européen des brevets**

(11) **EP 1 073 472 B1**

(12) **FASCICULE DE BREVET EUROPEEN**

(45) Date de publication et mention
de la délivrance du brevet:
**20.11.2002 Bulletin 2002/47**

(51) Int Cl.[7]: **A61K 49/00, A61K 9/08**

(21) Numéro de dépôt: **99913060.2**

(22) Date de dépôt: **22.04.1999**

(86) Numéro de dépôt international:
**PCT/CH99/00163**

(87) Numéro de publication internationale:
**WO 99/053962 (28.10.1999 Gazette 1999/43)**

(54) **SOLUTION D'UN ESTER DE L' ALA POUR LE DIAGNOSTIC OU LE TRAITEMENT DE LESIONS TISSULAIRES**

E-ALA ENTHALTENDE LÖSUNG FÜR DIE DIAGNOSE ODER THERAPIE VON GEWEBESCHÄDEN

SOLUTION CONTAINING AN ESTER OF ALA FOR DIAGNOSING OR TREATING TISSUE PATHOLOGIES

(84) Etats contractants désignés:
**AT BE CH DE DK ES FI FR GB GR IE IT LI LU MC NL PT SE**

(30) Priorité: **22.04.1998 FR 9805425**

(43) Date de publication de la demande:
**07.02.2001 Bulletin 2001/06**

(73) Titulaires:
• **Marti, Alexandre**
  **1209 Genève (CH)**
• **Lange, Norbert**
  **1004 Lausanne (CH)**
• **Zellweger, Matthieu**
  **1007 Lausanne (CH)**
• **Wagnieres, George**
  **1110 Morges (CH)**
• **Van den Bergh, Hubert**
  **1376 Goumoens-la-Ville (CH)**
• **Jichlinski, Patrice**
  **1052 Le Mont-sur-Lausanne (CH)**
• **Kucera, Pavel**
  **1000 Lausanne 27 (CH)**

(72) Inventeurs:
• **Marti, Alexandre**
  **1209 Genève (CH)**
• **Lange, Norbert**
  **1004 Lausanne (CH)**
• **Zellweger, Matthieu**
  **1007 Lausanne (CH)**
• **Wagnieres, George**
  **1110 Morges (CH)**

• **Van den Bergh, Hubert**
  **1376 Goumoens-la-Ville (CH)**
• **Jichlinski, Patrice**
  **1052 Le Mont-sur-Lausanne (CH)**
• **Kucera, Pavel**
  **1000 Lausanne 27 (CH)**

(74) Mandataire: **Nithardt, Roland et al**
  **Cabinet Roland Nithardt,**
  **Conseils en Propriété Industrielle S.A.,**
  **Y-Parc,**
  **Rue Galilée 9**
  **1400 Yverdon-les-Bains (CH)**

(56) Documents cités:
**WO-A-96/28412**

• **FINK-PUCHES ET AL: "Primary clinical response and long-term follow-up of solar keratoses treated with topically applied 5-aminolevulinic acid and irradiation by different wave bands of light" JOURNAL OF PHOTOCHEMISTRY AND PHOTOBIOLOGY B: BIOLOGY, vol. 41, 1997, pages 145-151, XP002078603**

• DATABASE EMBASE [Online] ELSEVIER SCIENCE PUBLISHERS, AMSTERDAM, NL AN=96195268, THOMAS P.: "[Topical photodynamic therapy]. PHOTOTHERAPIE DYNAMIQUE TOPIQUE." XP002091084 & NOUVELLES DERMATOLOGIQUES, (1996) 15/5 (407-411). ISSN: 0752-5370 CODEN: NODEE2, France

• DATABASE MEDLINE [Online] US NATIONAL LIBRARY OF MEDICINE (NLM), BETHESDA, MD, US AN=97346991, CHANG S C ET AL: "The efficacy of an iron chelator (CP94) in increasing cellular protoporphyrin IX following intravesical 5-aminolaevulinic acid administration: an in vivo study." XP002091085 & JOURNAL OF PHOTOCHEMISTRY AND PHOTOBIOLOGY. B, BIOLOGY, (1997 APR) 38 (2-3) 114-22. JOURNAL CODE: JLI. ISSN: 1011-1344., Switzerland

## Description

### Domaine technique

La présente invention concerne une solution d'un ester d'acide 5-aminolévulinique (E-ALA) pour la réalisation d'une préparation pharmaceutique utilisable pour le diagnostic et/ou le traitement de lésions tissulaires et/ou cellulaires par irradiation locale au moyen d'un rayonnement émis par une source d'énergie lumineuse suivie, dans le cas du diagnostic, d'une détection de la fluorescence émise par des substances dont l'acide 5-aminolévulinique (ALA) ou l'E-ALA sont des précurseurs, en particulier la protoporphyrine IX (PpIX).

### Technique antérieure

On connaît déjà le principe de l'utilisation de composés dont l'ALA, ou les esters d'ALA (E-ALA) et notamment l'hydrochlorure d'hexylester d'ALA (h-ALA) sont des précurseurs, pour faire un diagnostic et/ou un traitement de lésions, en particulier de lésions cancéreuses. Ce principe est bien décrit dans la demande de brevet publiée sous le No WO 96/28412. L'administration de la solution peut être faite par voie orale ou par voie parentérale, par exemple sous forme d'une injection intradermique, sous-cutanée, intrapéritonique ou intraveineuse. Cette administration peut aussi se faire de façon topique, par exemple locale, en exposant la surface de l'organe à traiter à une solution d'E-ALA ou d'ALA. Un tampon imbibé d'une telle solution peut également être utilisé pour procéder à une administration topique. La concentration de la solution d'ester d'ALA (E-ALA) préconisée dans cette publication est comprise entre 1 et 50% et de préférence entre 15 et 30%. Cependant, cette concentration ne génère essentiellement pas de PpIX dans certains des organes principalement concernés par ce genre de traitement, notamment la vessie urinaire.

Dans les publications faites dans JOURNAL OF PHOTOCHEMISTRY AND PHOTOBIOLOGY B, BIOLOGY respectivement par Fink-Puches et al sous le titre "Primirary clinical response and long-term follow-up of solar keratoses treated with topically applied 5-aminolevulinic acid and irradiation by different wave bands of light", et Chang et al sous le titre "The efficacy of an iron chelator (CP94) in increasing cellular protoporphyrin IX following intravestical 5-aminolaevulinic acid administration: an in vivo study" ainsi que celle faite dans NOUVELLES DERMATOLOGIQUES par P. Thomas sous le titre "Photothérapie dynamique topique", le produit utilisé dans les traitements est de l'ALA et non pas un ester d'ALA, ce qui est très différent au niveau des concentrations. En effet, les concentrations d'ALA utilisées sont au minimum 45 à 60 fois supérieures à celles nécessaires dans le cas de l'utilisation d'une solution d'ester d'ALA (E-ALA).

L'administration de cette substance avec des concentrations aussi fortes s'est révélée, dans certains cas, toxique pour des tissus humains. Cette toxicité présente même en l'absence de rayonnement lumineux, peut contrarier gravement la génération de prptoporphyrine IX (PpIX). De ce fait, de telles concentrations ne peuvent, dans certains cas, pas être utilisées ou ne sont pas optimales pour permettre le dépistage et/ou le traitement de lésions.

En outre, les temps requis pour activer les principes actifs induits par la solution médicamenteuse se sont avérés extrêmement longs si l'acide 5-aminolévulinique libre, c'est-à-dire non estérifié (ALA) est utilisé. De ce fait, le diagnostic ainsi que le traitement, ne peuvent se faire avec de l'ALA libre qu'en milieu hospitalier, étant donné qu'ils requièrent souvent une immobilisation de longue durée, d'environ 5 heures, du patient.

Dans le cadre d'une tendance généralisée de réduction du coût des soins médicaux et du développement des soins à domicile, dans des cabinets privés ou des hôpitaux dits de jour, la démarche actuellement connue est lourde, contraignante pour le patient et coûteuse pour les assurances maladies et pour la communauté.

Malgré le progrès technologique que constitue l'application de l'ALA ou l'E-ALA pour diagnostiquer de façon précoce et traiter efficacement certaines affections, la généralisation de la méthode est ralentie par ces inconvénients majeurs.

### Exposé de l'invention

Le but de la présente invention est de pallier ces inconvénients en mettant au point une solution destinée au diagnostic et/ou au traitement de lésions cancéreuses, notamment dans le domaine de l'urologie, administrée à des concentrations qui ne portent pas préjudice à la biosynthèse des composés actifs, et qui démontre une grande efficacité lorsqu'elle est appliquée pendant des temps suffisants relativement courts pour autoriser un traitement sinon ambulatoire du moins en clinique de jour, voire en cabinet médical. Cette solution doit en particulier permettre une forte accumulation de la PpIX en un minimum de temps et une très bonne distribution de cette PpIX à travers les tissus traités.

Ce but est atteint par une solution d'un ester d'acide 5-aminolévulinique (E-ALA) telle que définie en préambule, caractérisée en ce que la concentration $\underline{C}$ de l'E-ALA dans la solution est inférieure à 1% et est comprise entre 0,01% et 0,5% (0,01% $\leq \underline{C} \leq$ 0,5%).

En effet, il s'est avéré que le fait d'utiliser une très faible concentration de E-ALA dans la solution augmente la synthèse de la PpIX et homogénéise la distribution dans les couches cellulaires tout en diminuant fortement la toxicité secondaire de la solution pour les cellules traitées. Ceci est d'autant plus important que lors d'un traitement d'une tumeur par photothérapie dynamique, en raison du photobleaching rapide qui réduit la concentration de PpIX, la destruction complète de cette tumeur sous-entend une accumulation initiale intracellulaire de PpIX élevée et une bonne distribution dans les couches tumorales.

De façon particulièrement avantageuse, l'ester d'ALA (E-ALA) donnant les meilleurs résultats est de l'hydrochlorure d'hexylester d'ALA (h-ALA).

De préférence, la solution est réalisée par dissolution d'ester d'ALA (E-ALA) dans un solvant compatible avec l'organisme humain ou animal.

Ledit solvant est avantageusement choisi parmi l'une des substances suivantes : eau filtrée stérilisée, solution physiologique de NaCl, solution tampon de phosphate, alcool.

Sous une forme préférentielle, la solution comprend un composant pour ajuster le PH à une valeur physiologique comprise entre 4,8 et 8,1.

Dans une forme de réalisation avantageuse, la solution peut comporter une substance complémentaire pour empêcher la transformation du PpIX en heme par complexage du fer dans les cellules vivantes.

Ladite substance complémentaire peut être un EDTA (tétra acétate diaminoéthylique), de la déferroxamine ou du desféral.

**Meilleure manière de réaliser l'invention**

La présente invention sera mieux comprise en référence à la description ci-dessous d'une forme de réalisation préférée de la solution selon l'invention et de ses variantes et, à titre d'illustration, d'une application particulièrement avantageuse de cette solution pour le diagnostic et/ou le traitement de lésions à l'intérieur d'une cavité de l'organisme humain ou animal, telle que la vessie.

Une solution d'ester de l'acide 5-aminolévulinique (E-ALA) est préparée par dissolution de cette substance, par exemple à l'état de poudre amorphe ou sous forme cristalline, dans un solvant approprié, compatible avec une utilisation in vivo. A titre d'exemple cette solution peut être de l'eau déminéralisée stérilisée, une solution de NaCl physiologique contenant approximativement 9% de Nacl, une solution tampon de phosphate, un alcool ou une solution contenant un alcool ou similaire.

Cette solution est de préférence ajustée en PH à une valeur dite physiologique qui dépend de l'application et principalement de l'organe à traiter concerné. Cette valeur du PH est habituellement comprise entre 4,8 et 8,1. Dans le cas d'une intervention à l'intérieur de la vessie, le PH est de préférence compris entre 5,3 et 7,4.

La solution peut être complétée par l'addition d'une substance complémentaire pour empêcher la transformation du PpIX en heme par complexage du fer dans les cellules vivantes. Cette substance complémentaire peut être un EDTA (tétra acétate diaminoéthylique), de la déferroxamine ou du desféral.

L'une des applications qui s'est révélée extrêmement intéressante est le diagnostic et le traitement de lésions du type cancéreuses dans le domaine urologique et en particulier sur les parois intérieures de la vessie.

Selon un mode d'application, l'administration de la solution peut être topique, en contact avec les parois intérieures de l'organe. La vessie est remplie d'environ 50ml de solution d'ester d'ALA (E-ALA) ou d'hexylester d'ALA (h-ALA) de faible concentration, à savoir une concentration $C$ (en poids) comprise entre 0,01% et 0,5% et de préférence égale à 0,2%.

L'instillation peut avoir une durée comprise entre ½ heure et 7 heures, mais de préférence comprise entre ½ heure et 4 heures.

On a constaté de façon surprenante que, sous ces faibles concentrations qui sont très différentes des concentrations de 15 à 30% actuellement utilisées dans ce même domaine, l'ester d'ALA (E-ALA) présente une plus grande efficacité, ce qui se mesure par une présence accrue de protoporphyrine IX (PpIX) fluorescente apparaissant aux emplacements des lésions sur les parois intérieures de la vessie et une meilleure distribution de cette protoporphyrine dans les couches cellulaires. En outre, grâce à ces faibles concentrations, la cytotoxicité est réduite, ce qui réduit considérablement les risques d'effets secondaires indésirables. En particulier, cette cytotoxicité réduite favorise la génération des substances photosensibles et/ou fluorescentes dont les E-ALA ou l'ALA libre sont des précurseurs. De plus, cette génération maximale de PpIX réduit le laps de temps séparant l'administration de la solution de l'intervention proprement dite.

Une variante du mode d'application peut être définie sous la dénomination "méthode topique fractionnée". Elle comprend par exemple les étapes suivantes :

- une première instillation de la vessie d'une durée de ½ heure à 3 heures et de préférence de l'ordre de 2 heures,
- un rinçage de la vessie,
- une deuxième instillation d'une durée de ½ heure à 3 heures, et de préférence de l'ordre de 2 heures,
- un rinçage de la vessie.

Après un délai d'attente compris entre 0 et 4 heures, et de préférence de l'ordre de 2 heures, le dépistage et/ou le traitement par fluorescence de la vessie peuvent avoir lieu.

L'application topique de la solution d'ester d'ALA (E-ALA) ou d'hexylester d'ALA (h-ALA) peut également être remplacée par une administration systémique. Dans ce cas, la solution est administrée par voie orale ou parentérale avec ou sans combinaison avec des composants appelés transporteurs, tels que par exemple le diméthylsulfoxyde, la glycine ou similaires, destinés à favoriser l'absorption et/ou la migration de la substance active, en l'occurrence l'ester d'ALA (E-ALA) ou d'hexylester d'ALA (h-ALA), par les tissus et/ou les cellules.

Enfin un moyen d'activation de la pénétration tissulaire ou cellulaire de l'ester d'ALA (E-ALA) ou d'hexylester d'ALA (h-ALA) peut consister à pratiquer une ionophorèse sur les parois de l'organe concerné.

Ces phases sont suivies d'une ou de plusieurs phases de traitement par photothérapie et/ou de traitement par fluorescence.

Lors d'un traitement par photothérapie, on irradie les parois de l'organe concerné, par exemple la vessie, avec un rayonnement lumineux appelé lumière excitatrice, monochromatique ou non, en continu ou de manière séquentielle, qui se situe de préférence dans le domaine spectral compris entre 300 et 900 nanomètres, et de préférence entre 350 et 650 nanomètres.

Pour procéder à ces photothérapies, l'éclairement E appliqué sur les parois de la vessie, qui est la puissance lumineuse par unité de surface, est compris entre 0,1 mW/cm$^2$ et 1W/cm$^2$, et de préférence entre 5mW/cm$^2$ et 500 mW/cm$^2$. Cette lumière induit une réaction phototoxique due à la présence de la protoporphyrine IX (PpIX) en particulier et/ou de ses photoproduits dans les tissus. Les doses d'éclairement peuvent être appliquées de façon homogène sur toute la paroi de l'organe ou de façon collimatée uniquement sur les sites qui ont été identifiés comme comportant des lésions.

Pour le diagnostic par fluorescence, on irradie les parois de la vessie au moyen d'un rayonnement dont la largeur spectrale est comprise entre 300 et 700 nanomètres, et de préférence entre 350 et 650 nanomètres. Pour procéder à ces dépistages par fluorescence, l'éclairement $\underline{E}$ appliqué sur les parois de la vessie (puissance lumineuse par unité de surface) est compris entre 1mW/cm$^2$ et 1W/cm$^2$ et de préférence entre 50mW/cm$^2$ et 500 mW/cm$^2$. Cette lumière excitatrice induit la fluorescence de substances dont l'E-ALA, et en particulier l'h-ALA, est un précurseur, en particulier de la PpIX. Cette fluorescence est collectée par un système optique et détectée à l'oeil, ou par un détecteur ponctuel, linéaire ou matriciel tel qu'une caméra.

L'utilisation de solutions à basses concentrations d'un ester d'ALA permet, en plus des avantages déjà mentionnés, d'aboutir à des faibles coûts d'une dose de produit que ce soit pour un traitement par photothérapie ou lors d'une photodétection, à de faibles coûts de production et à une pharmacie galénique simplifiée.

**Revendications**

1. Solution d'un ester d'acide 5-aminolévulinique (E-ALA) pour la réalisation d'une préparation pharmaceutique utilisable pour le diagnostic et/ou le traitement de lésions tissulaires et/ou cellulaires par irradiation locale au moyen d'un rayonnement émis par une source d'énergie lumineuse, suivie, dans le cas du diagnostic d'une détection de la fluorescence émise par des substances dont l'acide 5-aminolévulinique (ALA) ou l'E-ALA sont des précurseurs, en particulier la protoporphyrine IX (PpIX), **caractérisée en ce que** la concentration $\underline{C}$ de l'ester d'ALA (E-ALA) dans la solution est inférieure à 1% et comprise entre 0,01% et 0,5%.

$$0,01\% \leq \underline{C} \leq 0,5\%$$

2. Solution selon la revendication 1, **caractérisée en ce que** l'ester d'ALA (E-ALA) est de l'hexylester d'ALA (h-ALA).

3. Solution selon la revendication 1, **caractérisée en ce qu'**elle est réalisée par dissolution d'ester d'ALA dans un solvant compatible avec l'organisme humain ou animal.

4. Solution selon la revendication 3, **caractérisée en ce que** ledit solvant est choisi parmi l'une des substances suivantes : eau filtrée stérilisée, solution physiologique de NaCl, solution tampon de phosphate, alcool.

5. Solution selon la revendication 3, **caractérisée en ce qu'**elle comporte un composant pour ajuster le PH à une valeur physiologique comprise entre 4,8 et 8,1.

$$4,8 < PH < 8,1$$

6. Solution selon la revendication 1, **caractérisée en ce qu'**elle comporte une substance complémentaire pour empêcher la transformation du PpIX en heme par complexage du fer dans les cellules vivantes.

7. Solution selon la revendication 6, **caractérisée en ce que** ladite substance complémentaire est un EDTA (tétra acétate diaminoéthylique).

8. Solution selon la revendication 6, **caractérisée en ce que** ladite substance complémentaire est de la déferroxamine.

9. Solution selon la revendication 6, **caractérisée en ce que** ladite substance complémentaire est du desféral.

**Patentansprüche**

1. Lösung eines 5-Lävulinsäureester (E-ALS) zur Ausführung einer pharmazeutischen Zubereitung zur Diagnose und/oder zur Behandlung von Gewebeund/oder Zellschädigungen durch örtliche Bestrahlung mittels einer von einer Lichtquelle ausgehenden Strahlung, worauf im Falle der Diagnose eine Erkennung der Fluoreszenz folgt, die von Substanzen ausgegeben wird, von denen 5-Lävulinsäure (ALS) oder 5-Lävulinsäureester die Vorstufen sind, insbesondere Protoporphyrin IX (PpIX), **dadurch gekennzeichnet, daß** die Konzentration $\underline{C}$ des Lävulinsäureesters (E-ALS) in einer Lösung weniger als 1%, und zwischen einschließlich 0,01 % und 0,5 % beträgt.

$$0{,}01\ \% \le \underline{C} \le 0{,}5\ \%$$

2. Lösung nach Anspruch 1, **dadurch gekennzeichnet, daß** das Lävulinsäureester (E-ALS) Hexylester-Lävulinsäure (h-ALS) ist.

3. Lösung nach Anspruch 1, **dadurch gekennzeichnet, daß** sie durch die Auflösung von Lävulinsäureester in einer mit dem menschlichen oder tierischen Organismus kompatiblen Lösungsmittel ausgeführt wird.

4. Lösung nach Anspruch 3, **dadurch gekennzeichnet, daß** das Lösungsmittel aus den folgenden Substanzen ausgewählt wird: gefiltertes sterilisiertes Wasser, physiologische NaCl-Infusionslösung, Phosphat-Pufferlösung, Alkohol.

5. Lösung nach Anspruch 3, **dadurch gekennzeichnet, daß** sie eine Komponente zur Anpassung des PH-Wertes auf einen physiologischen Wert zwischen einschließlich 4,8 und 8,1 aufweist.

$$4{,}8 < pH < 8{,}1$$

6. Lösung nach Anspruch 1, **dadurch gekennzeichnet, daß** sie eine ergänzende Substanz aufweist, um die Umwandlung von Protoporphyrin IX in Häm durch Komplexierung von Eisen in lebendigen Zellen zu verhindern.

7. Lösung nach Anspruch 6, **dadurch gekennzeichnet, daß** die ergänzende Substanz eine EDTA (Ethylendiamintetra-Essigsäure) ist.

8. Lösung nach Anspruch 6, **dadurch gekennzeichnet, daß** die ergänzende Substanz Deferroxamin ist.

9. Lösung nach Anspruch 6, **dadurch gekennzeichnet, daß** die ergänzende Substanz Desferal ist.

## Claims

1. A solution of an ester of 5-aminolevulinic acid (E-ALA) for producing a pharmaceutical preparation used in the diagnosis and/or treatment of tissue and/or cell lesions with local irradiation using a beam emitted by a source of light energy, which is followed, in the case of diagnosis, of a detection of the fluorescence emitted by substances of which 5-aminolevulinic acid (ALA) or (E-ALA) acids are precursors, particularly protoporphyrin IX (PpIX), **characterized in that** the concentration $\underline{C}$ of the ester of ALA (E_ALA) in the solution is lower than 1% and ranges from 0.01% to 0.5 %.

$$0.01\% \le \underline{C} \le 0.5\%$$

2. A solution according to claim 1, **characterized in that** the ester of ALA (E-ALA) is a hexylester of ALA (h-ALA).

3. A solution according to claim 1, **characterized in that** the solution is produced by dissolving the ester of ALA in a solvent which is compatible with the human or animal organism.

4. A solution according to claim 3, **characterized in that** the said solvent is selected from one of the following substances : sterilized filtered water, physiological NaCl solution, phosphate buffer solution, alcohol.

5. A solution according to claim 3, **characterized in that** the solution comprises a component for adjusting the PH to a physiological value ranging from 4.8 to 8.1.

$$4.8 < PH < 8.1$$

6. A solution according to claim 1, **characterized in that** the solution comprises a complementary substance to prevent the transformation of the PpIX into a heme by iron complexing in the live cells.

7. A solution according to claim 6, **characterized in that** said complementary substance is a diaminoethyl tetra acetate (EDTA).

8. A solution according to claim 6, **characterized in that** said complementary substance is deferroxamine.

9. A solution according to claim 6, **characterized in that** said complementary substance is desferal.